# EUROPEAN PATENT SPECIFICATION

(11) **EP 3 533 863 B1**
(45) Date of publication and mention of the grant of the patent: **25.03.2026**
(21) Application number: 17864795.4
(22) Date of filing: 26.10.2017
(51) Int. Cl.: C12N 1/38, C12N 5/00, C12P 21/00, C12P 21/02

(54) **PROCESS FOR REDUCING UNWANTED CULTURE BYPRODUCTS IN CELL CULTURE MEDIUM**
VERFAHREN ZUR VERRINGERUNG UNERWÜNSCHTER KULTURNEBENPRODUKTE IN EINEM ZELLKULTURMEDIUM
PROCÉDÉ DE RÉDUCTION DE SOUS-PRODUITS DE CULTURE INDÉSIRABLES DANS UN MILIEU DE CULTURE CELLULAIRE

(30) Priority: 31.10.2016 KR 20160143427
(43) Date of publication of application: 04.09.2019
(73) Proprietor: Samsung Bioepis Co., Ltd., Incheon 21987 (KR)
(72) Inventor: PARK, Joon Tae, Seoul 03370 (KR); LEE, Kyung Ju, Incheon 21986 (KR); LIM, Joo Song, Anyang-si Gyeonggi-do 13960 (KR); LIM, Hyoung Taek, Incheon 21368 (KR); HUH, Yun Jeong, Suwon-si Gyeonggi-do 16703 (KR); LEE, Jae Sun, Incheon 21982 (KR); MIN, Hosung, Seoul 06515 (KR)
(74) Representative: Regimbeau
(86) International application number: PCT/KR2017/011872
(87) International publication number: WO 2018/080178

(56) References cited:
- EP-A1- 0 338 841
- EP-A1- 2 464 725
- KR-A- 20120 088 667
- KR-A- 20130 098 869
- KR-A- 20150 128 785
- US-A1- 2004 002 135
- YASHAS RAJENDRA ET AL: "Reduced glutamine concentration improves protein production in growth-arrested CHO-DG44 and HEK-293E cells", BIOTECHNOLOGY LETTERS, SPRINGER NETHERLANDS, DORDRECHT, vol. 34, no. 4, 30 November 2011 (2011-11-30), pages 619 - 626, XP035027584, ISSN: 1573-6776, DOI: 10.1007/S10529-011-0809-Z
- N. C. BOLS, R. C. GANASSIN, D. J. TOM, L. E. J. LEE: "Growth of fish cell lines in glutamine-free media", CYTOTECHNOLOGY, vol. 16, no. 3, 1994, pages 159 - 166, XP009513794, ISSN: 0920-9069, DOI: 10.1007/BF00749903

## Description

### TECHNICAL FIELD

The present disclosure relates to a process for reducing unwanted culture byproducts in a glutamine-free cell culture medium, and a culture process for maintaining or enhancing the productivity of a target protein.

### BACKGROUND ART

Animal cell culture in the biotechnology field has been considered to be an important technology for producing high value-added substances, such as new antibody production and vaccine development and production. Studies on cell metabolism and regulation methods also play an important role in raising the economic value of such substances. Thus, intensive research has been conducted on the components of media needed for culturing cells, and much effort has been made to increase productivity with respect to a target protein by adding various nutrients to the media components.

To increase the production efficiency of proteins including antibodies, it is important to improve cell viability. However, there are factors that limit the improvement of protein productivity. Among these factors, the most important factor is waste products or by-products generated during cell culture. These substances, which are unnecessarily produced in a cell metabolism process, may be toxic to cells or inhibit cell growth and metabolism in many different ways. The most representative example of these by-products is ammonia. Generally, there are many reports of cytotoxicity of 2 mM to 10 mM ammonia in a cell culture medium. For example, it is known that, in Chinese hamster ovary (CHO) cells, 8 mM ammonia inhibited cell growth by 50% (Kurano N, Leist C, et al., 1990, J. Biotechnol. 15:113-128), and inhibits glycosylation of proteins by directly inhibiting the expression of major enzymes involved in glycosylation (Chen P, and Harcum SW., 2006, Metab. Eng., 8:123-132). Accordingly, ammonia may not only simply cause cytotoxicity, but also cause even structural and functional defects of a target protein to be produced. In addition, ammonia may also decrease the functions of other components involved in metabolism, and exhibit unnecessary functions, such as the release of essential metabolites from cells. However, it is difficult to easily remove ammonia from a cell culture medium, and thus the concentration of ammonia in a medium may be a more crucial factor in the production of a target protein.

Therefore, there is a need to develop a method of suppressing or reducing the generation of by-products, especially ammonia, in animal cell culture, while also maintaining or enhancing the productivity of a target protein.

### DESCRIPTION OF EMBODIMENTS

### TECHNICAL PROBLEM

Provided is a culture process for reducing unwanted culture by-products when a target protein is produced in a glutamine-free cell culture medium.

Provided is a culture process for maintaining or enhancing the productivity of a target protein in a glutamine-free cell culture medium.

### SOLUTION TO PROBLEM

The invention is defined in the appended claims and any other aspects or embodiments set forth herein are for information only.

In particular, a first aspect of the present invention, according to an aspect of an embodiment relates to a culture process for reducing an amount of ammonia when a target protein is produced in a glutamine-free cell culture medium, the culture process comprising: culturing a mammalian cell in the glutamine-free cell culture medium under conditions allowing survival or proliferation of the cell, wherein the cell culture medium comprises hypoxanthine at a concentration of about 150 µM to about 750 µM and thymidine at a concentration of about 25 µM to about 200 µM.

In a second aspect, the present invention relates to the use of a glutamine-free cell culture medium for reducing an amount of ammonia when a target protein is produced in the glutamine-free cell culture medium, wherein the cell culture medium comprises hypoxanthine at a concentration of about 150 µM to about 750 µM and thymidine at a concentration of about 25 µM to about 200 µM. Furthermore, an unclaimed embodiment herein described relates to an improved culture process for reducing an unwanted culture by-product when a target protein is produced in a glutamine-free cell culture medium includes culturing mammalian cell in a glutamine-free cell culture medium under conditions allowing survival or proliferation of the cell, wherein the cell culture medium includes hypoxanthine at a concentration of about 20 µM to about 1,000 µM and thymidine at a concentration of about 2 µM to about 1,000 µM.

The term "glutamine (Gln or Q)" as used herein refers to the amino acid L-glutamine.

The term "cell culture medium" as used herein refers to a solution containing nutrients needed for culturing an animal eukaryotic cell. The term "cell culture medium" as used herein may be used interchangeably with the term "culture medium," "medium," or "growth medium." The medium includes a commercialized or prepared medium used in culturing the animal cell.

The term "glutamine-free cell culture medium" as used herein refers to a cell culture medium not containing glutamine. The glutamine-free cell culture medium also includes a cell culture medium that does not substantially contain glutamine.

The cell culture medium herein described includes hypoxanthine at a concentration of about 20 µM to about 1,000 µM and thymidine at a concentration of about 2 µM to about 1,000 µM. According to the claimed invention, the cell culture medium comprises hypoxanthine at a concentration of about 150 µM to about 750 µM and thymidine at a concentration of about 25 µM to about 200 µM. Hypoxanthine is a purine derivative and is a compound having the molecular formula C₅H₄N₄O. The hypoxanthine may be natural or synthesized hypoxanthine. The thymidine is a compound of pyrimidine deoxynucleoside. Thymidine is also referred to as deoxythymidine, deoxyribosylthymine, or thymine deoxyriboside. The thymidine may be natural or synthesized thymidine.

The cell culture medium herein described may include hypoxanthine at a concentration of about 50 µM to about 1,000 µM. For example, the cell culture medium may include hypoxanthine at a concentration of about 100 µM to about 1,000 µM, between greater than about 100 µM and about 1,000 µM or less, about 100 µM to about 900 µM, about 100 µM to about 800 µM, about 100 µM to about 700 µM, about 100 µM to about 600 µM, about 100 µM to about 500 µM, about 100 µM to about 400 µM, about 100 µM to about 300 µM, about 100 µM to about 200 µM, about 110 µM to about 950 µM, about 120 µM to about 900 µM, about 130 µM to about 850 µM, about 140 µM to about 800 µM, about 150 µM to about 750 µM, about 150 µM to about 700 µM, about 150 µM to about 650 µM, about 150 µM to about 600 µM, about 150 µM to about 550 µM, about 150 µM to about 500 µM, about 150 µM to about 450 µM, about 150 µM to about 350 µM, about 150 µM to about 300 µM, about 150 µM to about 250 µM, about 175 µM to about 225 µM, or about 200 µM. According to the claimed invention, the cell culture medium comprises hypoxanthine at a concentration of about 150 µM to about 750.

The cell culture medium herein described may include thymidine at a concentration of about 2 µM to about 500 µM. For example, the cell culture medium may include thymidine at a concentration of about 6 µM to about 500 µM, about 9 µM to about 500 µM, about 12 µM to about 500 µM, about 16 µM to about 500 µM, between greater than 16 µM and about 500 µM or less, about 18 µM to about 450 µM, about 18 µM to about 400 µM, about 18 µM to about 350 µM, about 18 µM to about 300 µM, about 20 µM to about 300 µM, about 20 µM to about 250 µM, about 20 µM to about 200 µM, about 20 µM to about 150 µM, about 25 µM to about 200 µM, about 25 µM to about 150 µM, about 25 µM to about 100 µM, about 25 µM to about 75 µM, about 25 µM to about 50 µM, about 25 µM to about 40 µM, about 30 µM to about 40 µM, about 30 µM to about 35 µM, or about 32 µM. According to the claimed invention, the cell culture medium comprises thymidine at a concentration of about 25 µM to about 200 µM.

The term "culture by-product" as used herein refers to a substance that is produced in the cell and secreted to a medium when the animal cell is cultured. The culture by-product may adversely affect protein expression, survival, proliferation, growth, differentiation, or function of cultured cell. The term "culture by-product" may be used interchangeably with the term "waste product."

According to the present invention, the unwanted culture by-product is ammonia. The amount of ammonia may be reduced by about 10% or more, as compared to that in a glutamine-free culture medium that does not substantially contain at least one of hypoxanthine and thymidine. For example, the amount of ammonia may be reduced by about 15% or more, about 20% or more, about 30% or more, about 40% or more, about 50% or more, about 60% or more, about 70% or more, about 80% or more, about 90% or more, about 99% or more, about 100%, about 10% to about 60%, about 10% to about 50%, about 10% to about 40%, about 20% to about 60%, about 20% to about 50%, about 20% to about 40%, about 20% to about 30%, about 30% to about 40%, about 40% to about 50%, about 50% to about 60%, about 60% to about 70%, about 70% to about 80%, about 80% to about 90%, or about 90% to about 100%.

The process includes culturing a mammalian cell in a glutamine-free cell culture medium under conditions allowing survival or proliferation of the cell.

The mammalian cell refers to a cell derived from mouse, rat, rabbit, dog, cat, sheep, cow, horse, monkey, chimpanzee, or human. The cell may be a cell line.

The cell is, for example, a cell selected from the group consisting of Chinese hamster ovary (CHO) cell, DG44 cell, human embryonic kidney (HEK) cell, NS0 cell, PER.C6 cell, HeLa cell, and Madin-Darby Canine Kidney (MDCK) cell. The CHO cell may be CHO K1 or CHO DUKX cell. The HEK cell may be HEK 293 cell.

The conditions allowing survival or proliferation of the cell may vary according to the type of a cell. For example, the cell may be cultured in the presence of a medium containing nutrients needed for proliferating the cell. The cell may be cultured at a temperature of about 25°C to about 42°C, about 25°C to about 40°C, about 30°C to about 40°C, about 30°C to about 37°C, or about 37°C. The cell may be cultured in the presence of air containing about 1% CO₂ to about 10% CO₂, or about 5% CO₂ to about 10% CO₂.

The culture may vary according to the type of a cell. The culture may be performed using a known method. The culture may be performed in a plate, a flask, or the like. The culture may be performed by adhering the cell to a substrate or floating the cell in a culture solution. The culture may be performed by subculture. The culture may be batch culture. The batch culture may be fed-batch culture. When the culture is performed, the cell culture medium may be periodically replaced with a fresh medium. The cell may be cultured for about 1 day or more, about 2 days or more, about 3 days or more, about 4 days or more, about 5 days or more, about 6 days or more, about 1 week or more, about 10 days or more, about 2 weeks or more, about 3 weeks or more, about 1 month or more, about 1 day to about 1 month, about 1 day to about 3 weeks, about 1 day to about 2 weeks, about 2 days to about 2 weeks, about 3 days to about 2 weeks, about 4 days to about 2 weeks, about 5 days to about 2 weeks, about 6 days to about 2 weeks, or about 1 week to about 2 weeks.

The cell maintained in the above-described process conditions may exhibit increased glucose consumption, as compared to that in a glutamine-free culture medium that does not substantially contain at least one of hypoxanthine and thymidine. Since a cell uses glucose as an energy source, an increase in glucose consumption of cell may mean an increase in metabolic activity of cell. The glucose consumption may be increased by about 10% or more as compared to glucose consumption in a glutamine-free culture medium that does not substantially contain at least one of hypoxanthine and thymidine. For example, the glucose consumption may be increased by about 15% or more, about 20% or more, about 30% or more, about 40% or more, about 50% or more, about 60% or more, about 70% or more, about 80% or more, about 90% or more, about 99% or more, about 100%, about 10% to about 60%, about 10% to about 50%, about 10% to about 40%, about 10% to about 30%, about 15% to about 60%, about 15% to about 50%, about 15% to about 40%, about 15% to about 30%, about 15% to about 20%, about 20% to about 30%, about 30% to about 40%, about 40% to about 50%, about 50% to about 60%, about 60% to about 70%, about 70% to about 80%, about 80% to about 90%, or about 90% to about 100%.

The cell maintained in the above-described process conditions may exhibit increased cell viability, as compared to that in a glutamine-free culture medium that does not substantially contain at least one of hypoxanthine and thymidine. The cell viability refers to the ability of cultured cell to survive, grow, or proliferate in a cultured environment, and the cell viability (%) may be calculated as a relative ratio of viable cell density in an experimental group to that in a negative control. The cell viability may be increased by about 3% or more, as compared to cell viability in a glutamine-free culture medium that does not substantially contain at least one of hypoxanthine and thymidine. For example, the cell viability may be increased by about 5% or more, about 8% or more, about 10% or more, about 15% or more, about 20% or more, about 30% or more, about 40% or more, about 50% or more, about 60% or more, about 70% or more, about 80% or more, about 90% or more, about 3% to about 50%, about 3% to about 40%, about 3% to about 30%, about 3% to about 20%, about 3% to about 10%, about 3% to about 5%, about 5% to about 50%, about 5% to about 40%, about 5% to about 30%, about 5% to about 20%, about 5% to about 10%, about 10% to about 15%, about 15% to about 20%, about 20% to about 30%, about 30% to about 40%, about 40% to about 50%, about 50% to about 60%, about 60% to about 70%, about 70% to about 80%, about 80% to about 90%, or about 90% to about 100%.

The cell maintained under the above-described process conditions may exhibit an increased expression level (titer) of a polypeptide, as compared to that in a glutamine-free culture medium that does not substantially contain at least one of hypoxanthine and thymidine. The term "expression level" as used herein may be used interchangeably with the term "titer." The expression level of a polypeptide of the cell may be represented as the concentration (g/L) of a protein in a cell culture medium obtained through the process. The expression level of a polypeptide of the cell may be increased by about 10% or more, as compared to an expression level of a polypeptide in a glutamine-free culture medium that does not substantially contain at least one of hypoxanthine and thymidine. For example, the expression level of a polypeptide of the cell may be increased by about 15% or more, about 20% or more, about 30% or more, about 40% or more, about 50% or more, about 60% or more, about 70% or more, about 80% or more, about 90% or more, about 99% or more, about 100%, about 10% to about 60%, about 10% to about 50%, about 10% to about 40%, about 15% to about 60%, about 15% to about 50%, about 15% to about 40%, about 15% to about 30%, about 15% to about 20%, about 20% to about 30%, about 30% to about 40%, about 40% to about 50%, about 50% to about 60%, about 60% to about 70%, about 70% to about 80%, about 80% to about 90%, or about 90% to about 100%.

The target protein refers to a protein to be obtained by culturing cell. The target protein may be a cytoplasmic polypeptide, a membranous polypeptide, or a secretable polypeptide. The target protein may be a recombinant protein. The recombinant protein may be selected from the group consisting of, for example, an antibody or an antigen-binding fragment thereof, immunoadhesin, transforming growth factor (TGF)-β superfamily signaling molecule, blood coagulation factor, anti-tumor necrosis factor receptor (TNFR) antibody, anti-human epidermal growth factor receptor 2 (HER2) antibody, and TNFR:Fc. The term "antibody" as used herein is used interchangeably with the term "immunoglobulin (Ig)." A complete antibody has a structure including two full-length light chains and two full-length heavy chains, wherein each light chain is linked to the heavy chain by disulfide bonds (SS-bond). The antibody may be, for example, IgA, IgD, IgE, IgG, or IgM. The antibody may be a monoclonal antibody or a polyclonal antibody. The antibody may be an animal-derived antibody, a mouse-human chimeric antibody, a humanized antibody, or a human antibody. The term "antigen-binding fragment" refers to a fragment of the whole immunoglobulin structure and a portion of a polypeptide containing an antigen-binding portion. For example, the antigen-binding fragment may be scFv, (scFv)₂, Fv, Fab, Fab', Fv F(ab')₂, or a combination thereof. The immunoadhesin refers to an antibody-like protein and a fusion protein of the Fc region of an immunoglobulin and the functional domain of a binding protein (e.g., receptors, ligand cell-adhesion molecules). The TGF-β superfamily signaling molecules may be, for example, TGF-β 1, TGF-β 2, or TGF-β 3. The blood coagulation factor refers to a factor involved in blood coagulation. The blood coagulation factor may be, for example, fibrinogen, prothrombin, tissue tromboplastin, factor V, factor VII, factor VIII, factor IX, factor X, factor XI, factor XII, factor XIII, a von Willebrand factor, prekallikrein, high-molecular-weight kininogen, fibronectin, antithrombin III, heparin cofactor II, protein C, protein S, protein Z, a protein Z-linked protease inhibitor, plasminogen, alpha 2-antiplasmin, a tissue plasminogen activator, urokinase, plasminogen activator inhibitor-1, plasminogen activator inhibitor-2, or a cancer procoagulant. The anti-TNFR antibody may be an antibody specifically binding to TNFR or an antigen-binding fragment thereof. The anti-HER2 antibody may be an antibody specifically binding to HER2 or an antigen-binding fragment thereof. The TNFR:Fc refers to a fusion protein of TNFR and an Fc region.

The target protein may be selected from the group consisting of abagovomab, abciximab, adalimumab, adecatumumab, alemtuzumab, altumomab, altumomab pentetate, anatumomab, anatumomab mafenatox, arcitumomab, atlizumab, basiliximab, bectumomab, ectumomab, belimumab, benralizumab, bevacizumab, brentuximab, canakinumab, capromab, capromab pendetide, catumaxomab, certolizumab, clivatuzumab tetraxetan, daclizumab, denosumab, eculizumab, edrecolomab, efalizumab, etaracizumab, ertumaxomab, fanolesomab, fontolizumab, gemtuzumab, girentuximab, golimumab, ibritumomab, igovomab, infliximab, ipilimumab, labetuzumab, mepolizumab, muromonab, muromonab-CD3, natalizumab, necitumumab, nimotuzumab, ofatumumab, omalizumab, oregovomab, palivizumab, panitumumab, ranibizumab, rituximab, satumomab, sulesomab, ibritumomab, ibritumomab tiuxetan, tocilizumab, tositumomab, trastuzumab, ustekinumab, visilizumab, votumumab, zalutumumab, brodalumab, anrukinzumab, bapineuzumab, dalotuzumab, demcizumab, ganitumab, inotuzumab, mavrilimumab, moxetumomab pasudotox, rilotumumab, sifalimumab, tanezumab, tralokinumab, tremelimumab, urelumab, adornase alfa, Rebif, becaplermin, alteplase, laronidase, alefacept, aflibercept, Raxibacumab, darbepoetin alfa, Becaplermin Concentrate, interferon beta-1b, Botulinum Toxin Type A, rasburicase, asparaginase, epoetin alfa, etanercept, agalsidase beta, interferon alfacon-1, interferon alfa-2a, anakinra, Botulinum Toxin Type B, pegfilgrastim, oprelvekin, filgrastim, denileukin diftitox, peginterferon alfa-2a, aldesleukin, dornase alfa, interferon beta-1a, becaplermin, reteplase, interferon alfa-2, tenecteplase, drotrecogin alfa, rilonacept, romiplostim, methoxypolyethylene glycol-epoetin beta, a C1 esterase inhibitor, idursulfase, alglucosidase alfa, abatacept, galsulfase, palifermin, and interferon gamma-1b.

The polypeptide may be a target protein.

According to an aspect of another embodiment, an improved culture process for reducing an unwanted culture by-product when a target protein is produced in a glutamine-free cell culture medium includes culturing mammalian cell in the glutamine-free cell culture medium under conditions allowing survival or proliferation of the cell, wherein the cell culture medium includes hypoxanthine at a concentration of about 150 µM to about 300 µM and thymidine at a concentration of about 25 µM to about 150 µM, and cell maintained under the above-described process conditions exhibit at least one of a decrease in the amount of ammonia, which is an unwanted culture by-product, an increase in glucose consumption of the cell, an increase in cell viability, and an increase in an expression level of a polypeptide of the cell, as compared to those in a glutamine-free culture medium that does not substantially contain at least one of hypoxanthine and thymidine.

The cell, the cell culture medium, the glutamine-free cell culture medium, the culture by-product, the unwanted culture by-product, the conditions allowing survival or proliferation of the cell, the culturing, the hypoxanthine, the thymidine, the ammonia, the glucose consumption, the cell viability, the polypeptide, and the expression level of a polypeptide of the cell are the same as described above.

The cell maintained under the above-described process conditions may exhibit at least one of a decrease in the amount of ammonia, which is an unwanted culture by-product, an increase in glucose consumption of the cell, an increase in cell viability, and an increase in an expression level of a polypeptide of the cell, as compared to those in a glutamine-free culture medium that does not substantially contain at least one of hypoxanthine and thymidine. For example, the cell may exhibit an about 10% or more decrease in the amount of ammonia, an about 10% or more increase in glucose consumption of the cell, an about 3% or more increase in the cell viability, an about 10% or more increase in an expression level of a polypeptide of the cell, or a combination thereof.

The process according to an embodiment and the process according to another embodiment may further include supplementing the cell culture medium with uridine. The uridine is a compound of pyrimidine nucleoside. The uridine may be natural or synthesized uridine. The amount of uridine in the cell culture medium may range from about 0.01 mM to about 100 mM, about 0.05 mM to about 95 mM, about 0.1 mM to about 90 mM, about 0.1 mM to about 85 mM, about 0.5 mM to about 80 mM, about 1 mM to about 80 mM, about 1 mM to about 75 mM, about 1 mM to about 70 mM, about 1 mM to about 65 mM, about 1 mM to about 60 mM, about 1 mM to about 55 mM, about 1 mM to about 50 mM, about 1 mM to about 45 mM, about 1 mM to about 40 mM, about 1 mM to about 35 mM, about 1 mM to about 30 mM, about 1 mM to about 25 mM, about 1 mM to about 20 mM, about 1.5 mM to about 20 mM, about 2 mM to about 20 mM, about 2 mM to about 15 mM, about 2 mM to about 10 mM, about 2.5 mM to about 10 mM, about 2.5 mM to about 9 mM, about 2.5 mM to about 8 mM, about 3 mM to about 8 mM, about 3 mM to about 7 mM, about 3.5 mM to about 7 mM, about 3.5 mM to about 6 mM, about 3.5 mM to about 5 mM, or about 4 mM. The supplementation may be performed by adding uridine to the cell culture medium at the initial stage of the culture or during the culture.

The process according to an embodiment and the process according to another embodiment may further include supplementing the cell culture medium with magnesium (Mg). The Mg may be a Mg ion or a Mg salt. The amount of Mg in the cell culture medium may range from about 0.01 mM to about 100 mM, about 0.01 mM to about 95 mM, about 0.01 mM to about 90 mM, about 0.01 mM to about 85 mM, about 0.01 mM to about 50 mM, about 0.05 mM to about 85 mM, about 0.05 mM to about 80 mM, about 0.1 mM to about 80 mM, about 0.1 mM to about 75 mM, about 0.1 mM to about 70 mM, about 0.1 mM to about 65 mM, about 0.1 mM to about 60 mM, about 0.1 mM to about 55 mM, about 0.1 mM to about 50 mM, about 0.2 mM to about 50 mM, about 0.2 mM about 45 mM, about 0.2 mM about 40 mM, about 0.2 mM to about 35 mM, about 0.4 mM to about 35 mM, about 0.4 mM to about 30 mM, about 0.4 mM to about 25 mM, about 0.4 mM to about 20 mM, about 0.5 mM to about 25 mM, about 0.6 mM to about 20 mM, about 0.6 mM to about 15 mM, about 0.6 mM to about 10 mM, about 0.6 mM to about 9 mM, about 0.6 mM to about 8 mM, about 0.6 mM to about 7 mM, about 0.6 mM to about 6 mM, about 0.6 mM to about 5 mM, about 0.8 mM to about 5 mM, about 0.8 mM to about 4 mM, about 0.8 mM to about 3 mM, about 0.8 mM to about 2 mM, about 0.8 mM to about 1.5 mM, about 0.8 mM to about 1.2 mM, or about 1 mM. The supplementation may be performed by adding magnesium to the cell culture medium at the initial stage of the culture or during the culture.

According to the present description an improved culture process for producing a target protein in a glutamine-free cell culture medium may include culturing mammalian cell in a glutamine-free cell culture medium under conditions allowing survival or proliferation of the cell, wherein the cell culture medium includes uridine at a concentration of about 0.01 mM to about 100 mM.

The cell, the cell culture medium, the glutamine-free cell culture medium, the target protein, the conditions allowing survival or proliferation of the cell, and the uridine are the same as described above.

The cell maintained in the cell culture medium may exhibit increased cell viability, as compared to that in a glutamine-free cell culture medium that does not contain uridine. The cell viability may be increased by about 3% or more, as compared to cell viability in the glutamine-free cell culture medium that does not contain uridine. For example, the cell viability may be increased by about 5% or more, about 8% or more, about 10% or more, about 15% or more, about 20% or more, about 30% or more, about 40% or more, about 50% or more, about 60% or more, about 70% or more, about 80% or more, about 90% or more, about 3% to about 50%, about 3% to about 40%, about 3% to about 30%, about 3% to about 20%, about 3% to about 10%, about 3% to about 5%, about 5% to about 50%, about 5% to about 40%, about 5% to about 30%, about 5% to about 20%, about 5% to about 10%, about 10% to about 15%, about 15% to about 20%, about 20% to about 30%, about 30% to about 40%, about 40% to about 50%, about 50% to about 60%, about 60% to about 70%, about 70% to about 80%, about 80% to about 90%, or about 90% to about 100%.

The cell maintained in the cell culture medium may exhibit an increased viable cell density (VCD), as compared to that in a glutamine-free culture medium that does not contain uridine. The VCD may be increased by about 5% or more, as compared to VCD in a glutamine-free culture medium that does not contain uridine. For example, the VCD may be increased by about 5% or more, about 10% or more, about 15% or more, about 20% or more, about 30% or more, about 40% or more, about 50% or more, about 60% or more, about 70% or more, about 80% or more, about 90% or more, about 3% to about 5%, about 5% to about 10%, about 5% to about 60%, about 5% to about 50%, about 5% to about 40%, about 5% to about 30%, about 5% to about 20%, about 10% to about 60%, about 10% to about 50%, about 10% to about 40%, about 10% to about 30%, about 10% to about 20%, about 10% to about 15%, about 20% to about 30%, about 30% to about 40%, about 40% to about 50%, about 50% to about 60%, about 60% to about 70%, about 70% to about 80%, about 80% to about 90%, or about 90% to about 100%.

The cell culture medium may further include hypoxanthine at a concentration of about 20 µM to about 1,000 µM and thymidine at a concentration of about 2 µM to about 1,000 µM. The cell maintained in the cell culture medium may exhibit a reduced amount of ammonia as an unwanted culture by-product of the cell culture medium, as compared to that in a glutamine-free culture medium that does not substantially contain at least one of hypoxanthine and thymidine. The amount of ammonia may be reduced by about 15% or more, about 20% or more, about 30% or more, about 40% or more, about 50% or more, about 60% or more, about 70% or more, about 80% or more, about 90% or more, about 99% or more, about 100%, about 10% to about 60%, about 10% to about 50%, about 10% to about 40%, about 20% to about 60%, about 20% to about 50%, about 20% to about 40%, about 20% to about 30%, about 30% to about 40%, about 40% to about 50%, about 50% to about 60%, about 60% to about 70%, about 70% to about 80%, about 80% to about 90%, or about 90% to about 100%, as compared to that in a glutamine-free culture medium that does not substantially contain at least one of hypoxanthine and thymidine.

According to the present description, an improved culture process for producing a target protein in a glutamine-free cell culture medium may include culturing mammalian cell in the glutamine-free cell culture medium under conditions allowing survival or proliferation of the cell, wherein the cell culture medium includes magnesium at a concentration of about 0.01 mM to about 100 mM.

The cell, the cell culture medium, the glutamine-free cell culture medium, the target protein, the conditions allowing survival or proliferation of the cell, and the magnesium are the same as described above.

The cell maintained in the cell culture medium may exhibit increased cell viability, as compared to that in a glutamine-free culture medium that does not contain magnesium. The cell viability may be increased by about 1% or more when compared to cell viability in a glutamine-free culture medium that does not contain magnesium. For example, the cell viability may be increased by about 1.5% or more, about 2% or more, about 3% or more, about 5% or more, about 10% or more, about 15% or more, about 20% or more, about 30% or more, about 40% or more, about 50% or more, about 60% or more, about 70% or more, about 80% or more, about 90% or more, about 1% to about 5%, about 1% to about 10%, about 1% to about 20%, about 1% to about 30%, about 1.5% to about 5%, about 1.5% to about 10%, about 1.5% to about 20%, about 1.5% to about 30%, about 3% to about 5%, about 5% to about 10%, about 5% to about 15%, about 10% to about 15%, about 15% to about 20%, about 20% to about 30%, about 30% to about 40%, about 40% to about 50%, about 50% to about 60%, about 60% to about 70%, about 70% to about 80%, about 80% to about 90%, or about 90% to about 100%.

The cell maintained in the cell culture medium may exhibit increased cell viable density, as compared to that in a glutamine-free culture medium that does not contain magnesium. The cell viable density may be increased by about 5% or more, as compared to cell viable density in a glutamine-free culture medium. For example, the cell viable density may be increased by about 10% or more, about 15% or more, about 20% or more, about 30% or more, about 40% or more, about 50% or more, about 60% or more, about 70% or more, about 80% or more, about 90% or more, about 5% to about 60%, about 5% to about 50%, about 5% to about 40%, about 5% to about 30%, about 5% to about 20%, about 5% to about 10%, about 10% to about 60%, about 10% to about 50%, about 10% to about 40%, about 10% to about 30%, about 10% to about 20%, about 10% to about 15%, about 20% to about 30%, about 30% to about 40%, about 40% to about 50%, about 50% to about 60%, about 60% to about 70%, about 70% to about 80%, about 80% to about 90%, or about 90% to about 100%.

The cell culture medium may further include hypoxanthine at a concentration of about 20 µM to about 1,000 µM and thymidine at a concentration of about 2 µM to about 1,000 µM. The cell maintained in the cell culture medium may exhibit a reduced amount of ammonia as an unwanted culture by-product of the cell culture medium, as compared to that in a glutamine-free culture medium that does not substantially contain at least one of hypoxanthine and thymidine. The amount of ammonia may be reduced by about 15% or more, about 20% or more, about 30% or more, about 40% or more, about 50% or more, about 60% or more, about 70% or more, about 80% or more, about 90% or more, about 99% or more, about 100%, about 10% to about 60%, about 10% to about 50%, about 10% to about 40%, about 20% to about 60%, about 20% to about 50%, about 20% to about 40%, about 20% to about 30%, about 30% to about 40%, about 40% to about 50%, about 50% to about 60%, about 60% to about 70%, about 70% to about 80%, about 80% to about 90%, or about 90% to about 100%, as compared to that in a glutamine-free culture medium that does not substantially contain at least one of hypoxanthine and thymidine.

The glutamine-free culture medium that does not substantially contain at least one of hypoxanthine and thymidine may contain hypoxanthine at a concentration of less than 100 µM, 90 µM or less, 80 µM or less, 70 µM or less, 60 µM or less, 50 µM or less, 40 µM or less, 30 µM or less, 20 µM or less, 10 µM or less, 5 µM or less, or 1 µM or less, or may be a hypoxanthine-free medium. The glutamine-free culture medium that does not substantially contain at least one of hypoxanthine and thymidine may contain thymidine at a concentration of less than 16 µM, 15 µM or less, 14 µM or less, 13 µM or less, 12 µM or less, 11 µM or less, 10 µM or less, 9 µM or less, 8 µM or less, 7 µM or less, 6 µM or less, 5 µM or less, 4 µM or less, 3 µM or less, 2 µM or less, or 1 µM or less, or may be a thymidine-free medium.

In the embodiment or the other embodiment, the culturing process may include conditions of at least one temperature change, conditions of at least one pH change, or a combination thereof.

The conditions of at least one temperature change are as follows: the cell may be grown in a medium at a first temperature for 3 days or more, the temperature may be changed to a second temperature, which is about 1 °C to about 8°C lower than the first temperature, and the cell may be maintained at the second temperature for about 2 days or more. The cell may be grown in a medium at a first temperature for about 3 days or more, about 4 days or more, about 1 week or more, about 2 days to about 1 week, or about 2 days to about 4 days. The cell may be maintained in a medium at a second temperature value for about 2 days or more, about 3 days or more, about 4 days or more, about 1 week or more, about 2 weeks or more, about 3 weeks or more, about 1 month or more, about 1 day to about 1 month, about 1 week to about 3 weeks, about 1 day to about 2 weeks, about 1 day to about 1 week, or about 1 day to about 4 days. The first temperature may range from about 30°C to about 42°C, about 32 °C to about 42°C, about 32 °C to about 40°C, about 34°C to about 40°C, about 36°C to about 40°C, or about 37°C. The second temperature may range from about 25 °C to about 41 °C, about 27°C to about 41 °C, about 27°C to about 40°C, about 30°C to about 40°C, about 30°C to about 37°C, about 30°C to about 35°C, about 30°C to about 33°C, or about 31°C to about 33°C. A difference between the first and second temperatures may range from about 1 °C to about 8°C, about 1 °C to about 7°C, about 1 °C to about 6°C, about 1 °C to about 5°C, about 1 °C to about 4°C, about 1 °C to about 3°C, or about 1 °C to about 2°C.

The conditions of at least one pH change are as follows: the cell may be grown in a medium at a first pH value for about 2 days or more, the pH may be changed to a second pH value, which is about 0.05 to about 1 lower than the first pH value, and the cell may be grown at the second pH value for about 1 day or more. The cell may be grown in a medium at a first pH value for about 2 days or more, about 3 days or more, about 4 days or more, about 1 week or more, about 2 days to about 1 week, or about 2 days to about 4 days. The cell may be maintained in a medium at a second pH value for about 1 day or more, about 2 days or more, about 3 days or more, about 4 days or more, about 1 week or more, about 2 weeks or more, about 3 weeks or more, about 1 month or more, about 1 day to about 1 month, about 1 day to about 3 weeks, about 1 day to about 2 weeks, about 1 day to about 1 week, or about 1 day to about 4 days. The first pH value may range from about pH 6.8 to about pH 7.5, about pH 6.8 to about pH 7.2, about pH 6.8 to about pH 7.0, or about pH 7.0 to about pH 7.2. The second pH value may range from about pH 6.0 to about pH 7.1, about pH 6.0 to about pH 7.0, about pH 6.0 to about pH 6.8, about pH 6.0 to about pH 6.6, about pH 6.0 to about pH 6.4, or about pH 6.0 to about pH 6.2. A difference between the first and second pH values may range from, for example, about pH 0.05 to about pH 1, about pH 0.05 to about pH 0.9, about pH 0.05 to about pH 0.8, about pH 0.05 to about pH 0.7, about pH 0.05 to about pH 0.6, about pH 0.05 to about pH 0.5, about pH 0.05 to about pH 0.4, about pH 0.05 to about pH 0.3, about pH 0.05 to about pH 0.2, about pH 0.05 to about pH 0.1, or about pH 0.05 to about pH 0.08.

In the embodiment or the other embodiment, the process may further include adding glucose to the glutamine-free cell culture medium such that the concentration of the glucose is maintained at about 2 g/L or more.

The concentration of the glucose in the cell culture medium may be maintained at about 2 g/L or more, about 2.5 g/L or more, about 3.0 g/L or more, about 3.5 g/L or more, about 4 g/L or more, about 4.5 g/L or more, about 5 g/L or more, about 5.5 g/L or more, about 6 g/L or more, about 7 g/L or less, about 2 g/L to about 7 g/L, about 2 g/L to about 6 g/L, about 2.5 g/L to about 5.5 g/L, about 3 g/L to about 5 g/L, about 3.5 g/L to about 5 g/L, or about 3.5 g/L to about 4.5 g/L. The glucose may be added to the cell culture medium during cell culture.

### ADVANTAGEOUS EFFECTS OF DISCLOSURE

The cell herein described may be cultured in a medium prepared by adding hypoxanthine, thymidine, uridine, magnesium, or a combination thereof to a glutamine-free medium, thereby reducing the production of ammonia due to the cell, increasing an expression level of a polypeptide of the cell, increasing a viable cell density, and increasing cell viability, and accordingly, the production of a target protein from the cell may be significantly increased.

### BRIEF DESCRIPTION OF DRAWINGS

FIG. 1 is a graph showing the concentration (mM) of ammonia in a cell culture medium (glutamine-free) with respect to culture time (days) of CHO cells in the presence of hypoxanthine and thymidine (■: 0 mM glutamine + 2x HT, ●: 0 mM glutamine + 1x HT).
FIG. 2 is a graph showing the concentration (g/L) of glucose in a cell culture medium (glutamine-free) with respect to culture time (days) of CHO cells in the presence of hypoxanthine and thymidine (■: 0 mM glutamine + 2x HT, ●: 0 mM glutamine + 1x HT).
FIG. 3 is a graph showing cell viability (%) with respect to culture time (days) of CHO cells in the presence of hypoxanthine and thymidine (■: 0 mM glutamine + 2x HT, ●: 0 mM glutamine + 1x HT). FIG. 4 is a graph showing a polypeptide expression level (g/L) with respect to culture time (days) of CHO cells in the presence of hypoxanthine and thymidine ( 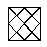 : 0 mM glutamine + 2x HT, 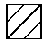 : 0 mM glutamine + 1x HT).
FIG. 5A is a graph showing viable cell density (CHO cells/ml) with respect to culture time (days) when 4 mM uridine was added to a 2x HT medium (■: 0 mM glutamine + 2x HT + 4 mM uridine, ●: 0 mM glutamine + 2x HT).
FIG. 5B is a graph showing viable cell density (CHO cells/ml) with respect to culture time (days) when 8 mM galactose was added to a 2x HT medium (■: 0 mM glutamine + 2x HT + 8 mM galactose, ●: 0 mM glutamine + 2x HT).
FIG. 6A is a graph showing cell viability (%) with respect to culture time (days) when 4 mM uridine was added to a 2x HT medium (■: 0 mM glutamine + 2x HT + 4 mM uridine, ●: 0 mM glutamine + 2x HT).
FIG. 6B is a graph showing cell viability (%) with respect to culture time (day) when 8 mM galactose was added to a 2x HT medium (■: 0 mM glutamine + 2x HT + 8 mM galactose, ●: 0 mM glutamine + 2x HT).
FIGS. 7 and 8 are graphs respectively showing viable cell density (CHO cells/ml) and cell viability (%) when 1 mM magnesium was added to a 2x HT medium (■: 0 mM glutamine + 2x HT + 1 mM magnesium, ●: 0 mM glutamine + 2x HT).

### MODE OF DISCLOSURE

Hereinafter, the present disclosure will be described in further detail with reference to the following examples. However, these examples are provided for illustrative purposes only and are not intended to limit the scope of the present disclosure.

### Example 1. Changes in Concentrations of Ammonia and Glucose in Cell Culture Medium according to the Presence of 2x HT

When cells are cultured, the concentration of ammonia in a cell culture medium is increased, and ammonia has cytotoxicity. In addition, during cell culture, as the number of cells increases, the amount of glucose consumed by the cells increases, and the content of glucose in the cell culture medium decreases. It was examined whether the amount of ammonia produced by cells during cell culture was decreased and the consumption of glucose of the cells was increased, in a case in which 2x HT was added to a cell culture medium.

First, a cell line expressing a fusion protein (CHO cell line) was prepared (the cell line was prepared by transfecting a CHO cell line with a vector expressing the fusion protein), and the cells were cultured in a glutamine-free medium. Cells at a density of 9 x 10⁵ cells/ml were inoculated in a cell culture dish, and a glutamine-free medium was added thereto. The medium was supplemented with a 100X stock (catalog number: 11067030, Gibco) of 10 mM sodium hypoxanthine and 1.6 mM thymidine so that the final concentrations of hypoxanthine and thymidine in the medium reached 100 µM and 16 µM, respectively (hereinafter, referred to as "1x HT"), or 200 µM and 32 µM, respectively (hereinafter, referred to as "2x HT"). The cells were cultured at 37°C and 5% CO₂, and the medium was replaced with a fresh medium every two days.

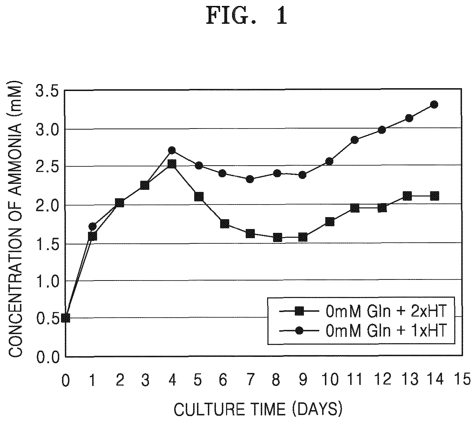

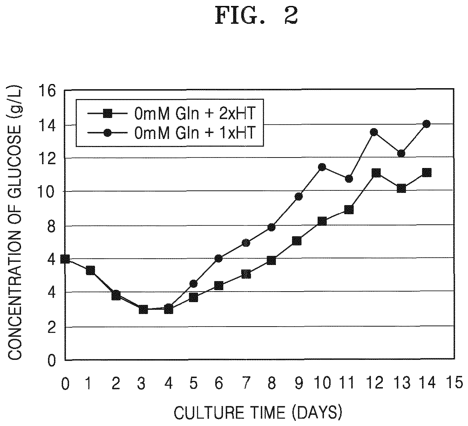

While the cells were cultured for about 15 days, the concentrations of ammonia (mM) and glucose (g/L) in the medium according to culture time (day) were measured using an ammonium ion-selective electrode and a glucose membrane kit (catalog No.: 24458, Nova Biomedical) of a Bioprofile analyzer (Model No.: BioProfile 400, Nova Biomedical). The results thereof are illustrated in FIGS. 1 and 2, respectively.

As illustrated in FIG. 1, when 2x HT was added, the concentration of ammonia in the cell culture medium was significantly decreased over cell culture time, as compared to when 1x HT was added. For example, 14 days after the culture, the concentration of ammonia in the medium was 3.31 mM when 1x HT was added, whereas the concentration of ammonia in the medium was decreased to 2.21 mM when 2x HT was added.

In addition, as illustrated in FIG. 2, when 2x HT was added to the glutamine-free medium, the concentration of glucose in the cell culture medium was significantly decreased over cell culture time, as compared to when 1x HT was added to the glutamine-free medium. For example, 14 days after the culture, the concentration of glucose in the medium was 14.00 g/L when 1x HT was added, whereas the concentration of glucose in the medium was decreased to 11.12 g/L when 2x HT was added.

Thus, it was confirmed that, when cells were cultured in a glutamine-free and 2x HT-containing medium, the production of ammonia by the cells was reduced, and cytotoxicity due to the produced ammonia was reduced, and accordingly, glucose consumption of the cells was increased, as compared to a case in which cells were cultured in a glutamine-free and 1x HT-containing medium.

### Example 2. Changes in Cell Viability and Polypeptide Expression Level of Cells according to Presence of 2x HT

It was examined whether there were differences in cell viability and polypeptide expression level (titer) of the cultured cells when the cells were cultured in a glutamine-free and 2x HT-containing medium, as compared to when the cells were cultured in a glutamine-free and 1x HT-containing medium.

The cells were cultured in a glutamine-free and 2x HT-containing medium or a glutamine-free and 1x HT-containing medium using the same method as that used in Example 1. Cells cultured in a glutamine-free medium were used as a negative control.

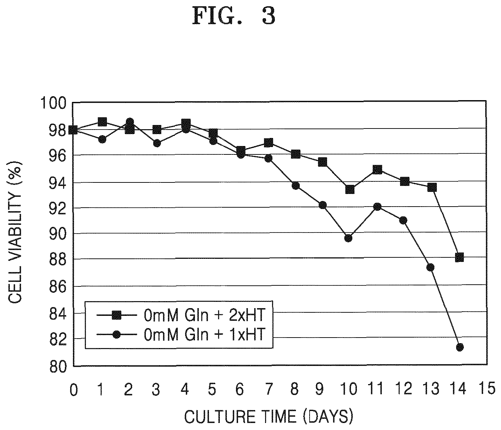

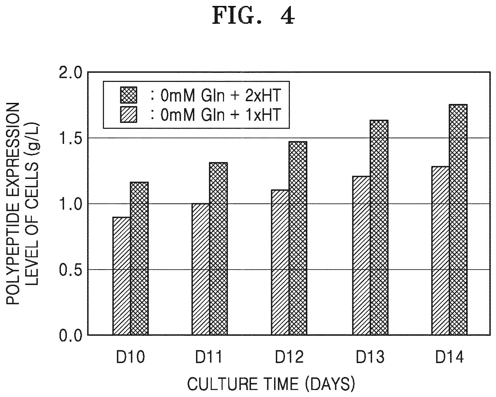

The relative cell viability (%) and polypeptide expression level (g/L) of the cells with respect to the negative control were measured using a Cedex HiRes analyzer (Model No.: 05650216001, Roche) and HPLC (Model No.: 720000370EN, Waters), respectively through a Trypan blue exclusion method and a UV280 measurement method. The results thereof are illustrated in FIGS. 3 and 4, respectively.

As illustrated in FIGS. 3 and 4, when cells were cultured in a glutamine-free and 2x HT-containing medium, the cell viability and polypeptide expression level of the cells were significantly increased, as compared to when cells were cultured in a glutamine-free and 1x HT-containing medium. For example, 14 days after the culture, cell viability was 81.4% when 1x HT was added, whereas the concentration of glucose in the medium was increased to 88.1% when 2x HT was added. In addition, 14 days after the culture, the polypeptide expression level of the cells was 1.29 g/L when 1x HT was added, whereas the concentration of glucose in the medium was increased to 1.76 g/L when 2x HT was added.

### Example 3. Effect of Uridine Addition on Viable Cell Density and Cell Viability

It was examined whether the addition of uridine affected viable cell density (VCD) and cell viability, when cells were cultured in a medium prepared by adding uridine to 2x HT.

Cells were prepared using the same method as that used in Example 1, and a glutamine-free and 2x HT-containing medium was added to the prepared cells. 4 mM of uridine (catalog No.: U3003, Sigma) or 8 mM of galactose (Catalog No.: G0750, Sigma) was added to the medium. The cells were cultured at 37°C and 5% CO₂, and the medium was replaced with a fresh medium every two days. Cells cultured in a glutamine-free medium were used as a negative control.

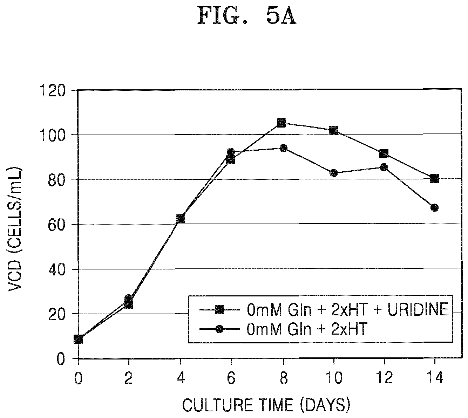

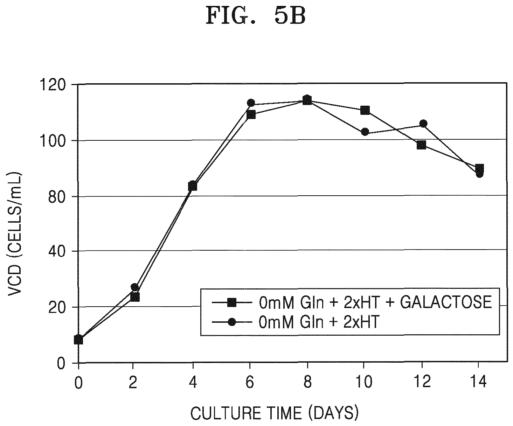

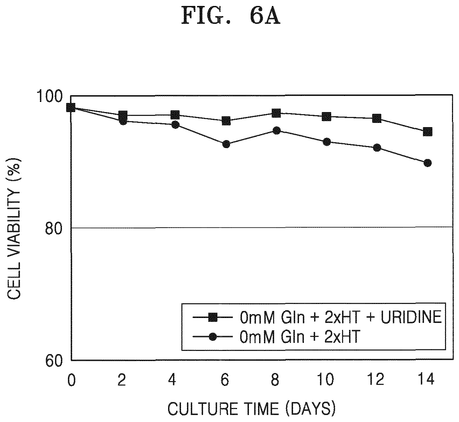

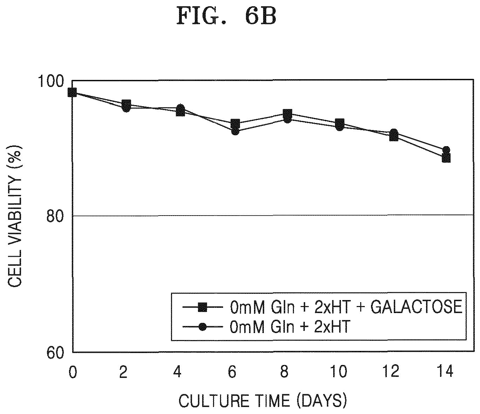

After about 14 days, viable cell density and cell viability were measured using a Cedex HiRes analyzer (Model No.: 05650216001, Roche) through a scanner-based imaging method and a Trypan blue exclusion method. The viable cell density (CHO cells/ml) of cells cultured in the presence of uridine and in the presence of galactose is illustrated in FIGS. 5A and 5B, respectively. In addition, the cell viability (%) of cells cultured in the presence of uridine and in the presence of galactose is illustrated in FIGS. 6A and 6B, respectively.

As illustrated in FIG. 5A, when uridine was added to a glutamine-free and 2x HT-containing medium, the viable cell density of the cultured cells was significantly increased, as compared to a case in which uridine was not added. For example, 14 days after the culture, the viable cell density was 67.22 cells/mL in the case in which the medium did not contain uridine, whereas the viable cell density was increased to 79.37 cells/mL in the case in which the medium contained uridine.

In contrast, as illustrated in FIG. 5B, there was no significant difference in viable cell density between a case in which galactose was added to a glutamine-free and 2x HT-containing medium and a case in which galactose was not added to the medium.

As illustrated in FIG. 6A, the viability of the cultured cells was significantly increased in a case in which uridine was added to a glutamine-free and 2x HT-containing medium, as compared to a case in which uridine was not added to the medium. For example, 14 days after the culture, the cell viability was 89.6% in the case in which the medium did not contain uridine, whereas the cell viability was increased to 94.6% in the case in which the medium contained uridine.

In contrast, as illustrated in FIG. 6B, there was no significant difference in viable cell density of the cultured cells between a case in which galactose was added to a glutamine-free and 2x HT-containing medium and a case in which galactose was not added to the medium.

Thus, it was confirmed that, when cells were cultured in a 2x HT- and uridine-containing medium, viable cell density and cell viability were significantly increased.

### Example 4. Effect of Magnesium Addition on Viable Cell Density and Cell Viability

Similar to Example 3, it was examined whether the addition of magnesium affected viable cell density and cell viability, when cells were cultured in a medium prepared by adding magnesium to 2x HT.

Cells were prepared using the same method as that used in Example 1, and a glutamine-free and 2x HT-containing medium was added to the prepared cells. 1 mM of magnesium (Catalog No.: M4880, Sigma) was added to the medium. The cells were cultured at 37°C and 5% CO₂, and the medium was replaced with a fresh medium every two days. Cells cultured in a glutamine-free medium were used as a negative control.

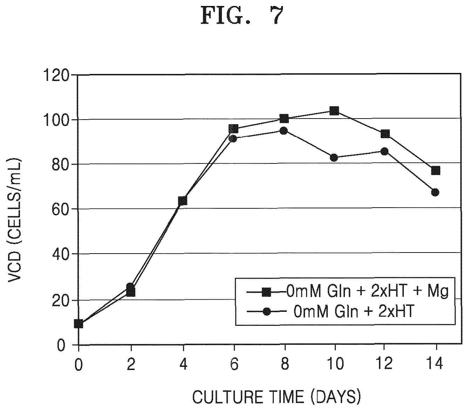

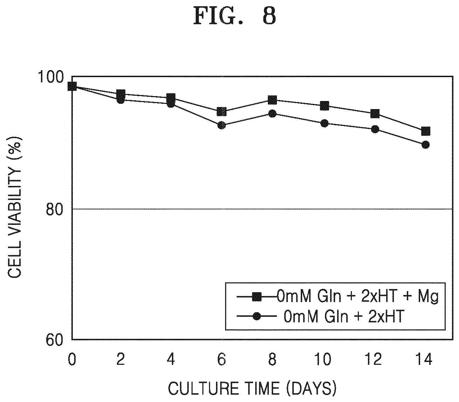

After about 14 days, viable cell density and cell viability were measured using a Cedex HiRes analyzer (Model No.: 05650216001, Roche) through a scanner-based imaging method and a Trypan blue exclusion method, respectively. The viable cell density (CHO cells/ml) and cell viability of the cells cultured in the presence of magnesium are illustrated in FIGS. 7 and 8, respectively.

As illustrated in FIGS. 7 and 8, the viable cell density of the cultured cells was significantly increased and the cell viability was significantly increased, in the case in which magnesium was added to the glutamine-free and 2x HT-containing medium, as compared to the case in which magnesium was not added to the medium. For example, 14 days after the culture, the viable cell density was 67.22 cells/mL in the case in which the medium did not contain magnesium, whereas the viable cell density was increased to 76.35 cells/mL in the case in which the medium contained magnesium. In addition, 14 days after the culture, the cell viability was 89.6% in the case in which the medium did not contain magnesium, whereas the cell viability was increased to 91.5% in the case in which the medium contained magnesium.

Thus, it was confirmed that the viable cell density and the cell viability were significantly increased in a case in which cells were cultured in a 2x HT- and magnesium-containing medium.

## Claims

1. A culture process for reducing an amount of ammonia when a target protein is produced in a glutamine-free cell culture medium, the culture process comprising:
culturing a mammalian cell in the glutamine-free cell culture medium under conditions allowing survival or proliferation of the cell,
wherein the cell culture medium comprises hypoxanthine at a concentration of about 150 µM to about 750 µM and thymidine at a concentration of about 25 µM to about 200 µM.

2. The culture process of claim 1, wherein an amount of the ammonia is reduced by about 10% or more, as compared to that in a glutamine-free culture medium that does not comprise at least one of hypoxanthine and thymidine.

3. The culture process of any one of claims 1 and 2, wherein (a) the cell maintained in the process conditions exhibits increased glucose consumption, as compared to that in a glutamine-free culture medium that does not comprise at least one of hypoxanthine and thymidine,
(b) the cell maintained in the process conditions exhibits increased cell viability, as compared to that in a glutamine-free culture medium that does not comprise at least one of hypoxanthine and thymidine, or
(c) the cell maintained in the process conditions exhibits an increased expression level (titer) of a polypeptide, as compared to that in a glutamine-free culture medium that does not comprise at least one of hypoxanthine and thymidine.

4. The culture process of claim 3,
wherein (a) the glucose consumption is increased by about 10% or more, as compared to glucose consumption in a glutamine-free culture medium that does not comprise at least one of hypoxanthine and thymidine,
(b) the cell viability is increased by about 3% or more, as compared to cell viability in a glutamine-free culture medium that does not comprise at least one of hypoxanthine and thymidine, or
(c) the cell maintained in the process conditions exhibits an increased expression level (titer) of a polypeptide, as compared to that in a glutamine-free culture medium that does not comprise at least one of hypoxanthine and thymidine.

5. The culture process of any one of claims 1 to 4, further comprising supplementing the cell culture medium with at least one of uridine and magnesium.

6. The culture process of any one of claims 1 to 5, wherein the target protein is selected from (a) the group consisting of an antibody or an antigen-binding fragment thereof, immunoadhesin, transforming growth factor (TGF)-β superfamily signaling molecule, blood coagulation factor, anti-tumor necrosis factor receptor (TNFR) antibody, anti-human epidermal growth factor receptor 2 (HER2) antibody, and TNFR:Fc; or
(b). the group consisting of abagovomab, abciximab, adalimumab, adecatumumab, alemtuzumab, altumomab, altumomab pentetate, anatumomab, anatumomab mafenatox, arcitumomab, atlizumab, basiliximab, bectumomab, ectumomab, belimumab, benralizumab, bevacizumab, brentuximab, canakinumab, capromab, capromab pendetide, catumaxomab, certolizumab, clivatuzumab tetraxetan, daclizumab, denosumab, eculizumab, edrecolomab, efalizumab, etaracizumab, ertumaxomab, fanolesomab, fontolizumab, gemtuzumab, girentuximab, golimumab, ibritumomab, igovomab, infliximab, ipilimumab, labetuzumab, mepolizumab, muromonab, muromonab-CD3, natalizumab, necitumumab, nimotuzumab, ofatumumab, omalizumab, oregovomab, palivizumab, panitumumab, ranibizumab, rituximab, satumomab, sulesomab, ibritumomab, ibritumomab tiuxetan, tocilizumab, tositumomab, trastuzumab, ustekinumab, visilizumab, votumumab, zalutumumab, brodalumab, anrukinzumab, bapineuzumab, dalotuzumab, demcizumab, ganitumab, inotuzumab, mavrilimumab, moxetumomab pasudotox, rilotumumab, sifalimumab, tanezumab, tralokinumab, tremelimumab, urelumab, adornase alfa, Rebif, becaplermin, alteplase, laronidase, alefacept, aflibercept, Raxibacumab, darbepoetin alfa, becaplermin concentrate, interferon beta)-1b, Botulinum toxin type A, rasburicase, asparaginase, epoetin alfa, etanercept, agalsidase beta, interferon alfacon-1, interferon alfa-2a, anakinra, Botulinum toxin type B, pegfilgrastim, oprelvekin, filgrastim, denileukin diftitox, peginterferon alfa-2a, aldesleukin, dornase alfa, interferon beta-1a, becaplermin, reteplase, interferon alfa-2, tenecteplase, drotrecogin alfa, rilonacept, romiplostim, methoxypolyethylene glycol-epoetin beta, a C1 esterase inhibitor, idursulfase, alglucosidase alfa, abatacept, galsulfase, palifermin, and interferon gamma-1b.

7. The culture process of any one of claims 5 and 6, wherein the cell maintained in the cell culture medium exhibits increased cell viability, preferably by about 3% or more, as compared to that in a glutamine-free culture medium that does not comprise at least one of uridine and magnesium.

8. The cultured process of claim 7, wherein the cell maintained in the cell culture medium exhibit an increased viable cell density, preferably by about 5% or more, as compared to that in a glutamine-free culture medium that does not comprise at least one of uridine and magnesium.

9. The culture process of any one of claims 1 to 8, wherein the cell is selected from the group consisting of CHO cell, DG44 cell, HEK cell, NS0 cell, PER.C6 cell, HeLa cell, and MDCK cell.

10. The culture process of any one of claims 1 to 9, wherein the culturing comprises conditions of at least one temperature change, conditions of at least one pH change, or a combination thereof,
wherein the conditions of at least one temperature change comprise the following processes: a cell is grown in a medium at a first temperature for 3 days or more, the temperature is changed to a second temperature, the second temperature being about 1 °C to about 8°C lower than the first temperature, and the cell is maintained at the second temperature for about 2 days or more, and
wherein the conditions of at least one pH change comprise the following processes: a cell is grown in a medium at a first pH value for about 2 days or more, the pH is changed to a second pH value, the second pH value being about 0.05 to about 1 lower than the first pH value, and the cell is grown at the second pH value for about 1 day or more.

11. The culture process of any one of claims 1 to 10, further comprising adding glucose to the glutamine-free cell culture medium such that a concentration of the glucose is maintained at about 2 g/L or more.

12. Use of a glutamine-free cell culture medium for reducing an amount of ammonia when a target protein is produced in the glutamine-free cell culture medium,
wherein the cell culture medium comprises hypoxanthine at a concentration of about 150 µM to about 750 µM and thymidine at a concentration of about 25 µM to about 200 µM.

## Patentansprüche

1. Kulturverfahren zur Reduzierung der Menge von Ammoniak bei der Produktion eines Zielproteins in einem glutaminfreien Zellkulturmedium, wobei das Kulturverfahren Folgendes umfasst:
Kultivieren einer Säugetierzelle in dem glutaminfreien Zellkulturmedium unter Bedingungen, die das Überleben oder die Proliferation der Zelle ermöglichen,
wobei das Zellkulturmedium Hypoxanthin in einer Konzentration von etwa 150 µM bis etwa 750 µM und Thymidin in einer Konzentration von etwa 25 µM bis etwa 200 µM umfasst.

2. Kulturverfahren nach Anspruch 1, wobei die Menge des Ammoniaks um etwa 10 % oder mehr reduziert wird, im Vergleich zu der in einem glutaminfreien Kulturmedium, das nicht mindestens eines von Hypoxanthin und Thymidin umfasst.

3. Kulturverfahren nach einem der Ansprüche 1 und 2, wobei (a) die unter den Verfahrensbedingungen gehaltene Zelle im Vergleich zu der in einem glutaminfreien Kulturmedium, das nicht mindestens eines von Hypoxanthin und Thymidin umfasst, einen erhöhten Glukoseverbrauch aufweist,
(b) die unter den Verfahrensbedingungen gehaltene Zelle im Vergleich zu der in einem glutaminfreien Kulturmedium, das nicht mindestens eines von Hypoxanthin und Thymidin umfasst, eine erhöhte Zellviabilität aufweist, oder
(c) die unter den Verfahrensbedingungen gehaltene Zelle im Vergleich zu der in einem glutaminfreien Kulturmedium, das nicht mindestens eines von Hypoxanthin und Thymidin umfasst, ein erhöhtes Expressionsniveau (Titer) eines Polypeptids aufweist.

4. Kulturverfahren nach Anspruch 3,
wobei (a) der Glukoseverbrauch um etwa 10 % oder mehr erhöht ist, im Vergleich zum Glukoseverbrauch in einem glutaminfreien Kulturmedium, das nicht mindestens eines von Hypoxanthin und Thymidin umfasst,
(b) die Zellviabilität um etwa 3 % oder mehr erhöht wird, im Vergleich zur Zellviabilität in einem glutaminfreien Kulturmedium, das nicht mindestens eines von Hypoxanthin und Thymidin umfasst, oder
(c) die unter den Verfahrensbedingungen gehaltene Zelle im Vergleich zu der in einem glutaminfreien Kulturmedium, das nicht mindestens eines von Hypoxanthin und Thymidin umfasst, ein erhöhtes Expressionsniveau (Titer) eines Polypeptids aufweist.

5. Kulturverfahren nach einem der Ansprüche 1 bis 4, das ferner das Ergänzen des Zellkulturmediums mit mindestens einem von Undin und Magnesium umfasst.

6. Kulturverfahren nach einem der Ansprüche 1 bis 5, wobei das Zielprotein ausgewählt ist aus (a) der Gruppe bestehend aus einem Antikörper oder einem Antigen-bindenden Fragment davon, Immunoadhesin, einem Signalmolekül der Transforming Growth Factor (TGF)-β-Superfamilie, einem Blutgerinnungsfaktor, einem Anti-Tumor-Nekrose-Faktor-Rezeptor(TNFR)-Antikörper, einem Anti-Human-Epidermal-Growth-Factor-Rezeptor-2(HER2)-Antikörper und TNFR:Fc; oder
(b). der Gruppe bestehend aus Abagovomab, Abciximab, Adalimumab, Adecatumumab, Alemtuzumab, Altumomab, Altumomab-Pentetat, Anatumomab, Anatumomab mafenatox, Arcitumomab, Atlizumab, Basiliximab, Bectumomab, Ectumomab, Belimumab, Benralizumab, Bevacizumab, Brentuximab, Canakinumab, Capromab, Capromab Pendetid, Catumaxomab, Certolizumab, Clivatuzumab Tetraxetan, Daclizumab, Denosumab, Eculizumab, Edrecolomab, Efalizumab, Etaracizumab, Ertumaxomab, Fanolesomab, Fontolizumab, Gemtuzumab, Girentuximab, Golimumab, Ibritumomab, Igovomab, Infliximab, Ipilimumab, Labetuzumab, Mepolizumab, Muromonab, Muromonab-CD3, Natalizumab, Necitumumab, Nimotuzumab, Ofatumumab, Omalizumab, Oregovomab, Palivizumab, Panitumumab, Ranibizumab, Rituximab, Satumomab, Sulesomab, Ibritumomab, Ibritumomab-Tiuxetan, Tocilizumab, Tositumomab, Trastuzumab, Ustekinumab, Visilizumab, Votumumab, Zalutumumab, Brodalumab, Anrukinzumab, Bapineuzumab, Dalotuzumab, Demcizumab, Ganitumab, Inotuzumab, Mavrilimumab, Moxetumomab-Pasudotox, Rilotumumab, Sifalimumab, Tanezumab, Tralokinumab, Tremelimumab, Urelumab, Adornase Alfa, Rebif, Becaplermin, Alteplase, Laronidase, Alefacept, Aflibercept, Raxibacumab, Darbepoetin alfa, Becaplermin-Konzentrat, Interferon beta-1b, Botulinumtoxin Typ A, Rasburicase, Asparaginase, Epoetin alfa, Etanercept, Agalsidase beta, Interferon Alfacon-1, Interferon alfa-2a, Anakinra, Botulinumtoxin Typ B, Pegfilgrastim, Oprelvekin, Filgrastim, Denileukin-Diftitox, Peginterferon alfa-2a, Aldesleukin, Dornase alfa, Interferon beta-1a, Becaplermin, Reteplase, Interferon alfa-2, Tenekteplase, Drotrecogin alfa, Rilonacept, Romiplostim, Methoxypolyethylenglykol-Epoetin Beta, einem C1-Esterase-Inhibitor, Idursulfase, Alglucosidase alfa, Abatacept, Galsulfase, Palifermin und Interferon gamma-1b.

7. Kulturverfahren eines der Ansprüche 5 und 6, wobei die Zelle, die im Zellkulturmedium gehalten wird, eine erhöhte Zellviabilität aufweist, vorzugsweise um etwa 3 % oder mehr, im Vergleich zu der in einem glutaminfreien Kulturmedium, das nicht mindestens eines von Uridin und Magnesium umfasst.

8. Kultiviertes Verfahren nach Anspruch 7, wobei die Zelle, die im Zellkulturmedium gehalten wird, eine erhöhte lebensfähige Zellendichte aufweist, vorzugsweise um etwa 5 % oder mehr, im Vergleich zu der in einem glutaminfreien Kulturmedium, das nicht mindestens eines von Undin und Magnesium umfasst.

9. Kulturverfahren nach einem der Ansprüche 1 bis 8, wobei die Zelle aus der Gruppe bestehend aus CHO-Zelle, DG44-Zelle, HEK-Zelle, NS0-Zelle, PER.C6-Zelle, HeLa-Zelle und MDCK-Zelle ausgewählt ist.

10. Kulturverfahren nach einem der Ansprüche 1 bis 9, wobei das Kultivieren Bedingungen mindestens einer Temperaturänderung, Bedingungen mindestens einer pH-Wert-Änderung oder eine Kombination davon umfasst,
wobei die Bedingungen für mindestens eine Temperaturänderung die folgenden Prozesse umfassen: eine Zelle wird 3 Tage oder länger bei einer ersten Temperatur in einem Medium gezüchtet, die Temperatur wird auf eine zweite Temperatur geändert, wobei die zweite Temperatur etwa 1 °C bis etwa 8 °C niedriger als die erste Temperatur ist, und die Zelle wird etwa 2 Tage oder länger bei der zweiten Temperatur gehalten, und
wobei die Bedingungen für mindestens eine pH-Wert-Änderung folgende Prozesse umfassen: eine Zelle wird für etwa 2 Tage oder länger bei einem ersten pH-Wert in einem Medium gezüchtet, der pH-Wert wird auf einen zweiten pH-Wert geändert, wobei der zweite pH-Wert etwa 0,05 bis etwa 1 niedriger als der erste pH-Wert ist, und die Zelle wird für etwa 1 Tag oder länger bei dem zweiten pH-Wert gezüchtet.

11. Kulturverfahren nach einem der Ansprüche 1 bis 10, ferner umfassend das Hinzufügen von Glukose zu dem glutaminfreien Zellkulturmedium, sodass eine Konzentration der Glukose von etwa 2 g/L oder mehr aufrechterhalten wird.

12. Verwendung eines glutaminfreien Zellkulturmediums zur Reduzierung der Menge von Ammoniak bei der Produktion eines Zielproteins im glutaminfreien Zellkulturmedium,
wobei das Zellkulturmedium Hypoxanthin in einer Konzentration von etwa 150 µM bis etwa 750 µM und Thymidin in einer Konzentration von etwa 25 µM bis etwa 200 µM umfasst.

## Revendications

1. Procédé de mise en culture pour réduire la quantité d'ammoniac lorsqu'une protéine cible est produite dans un milieu de culture cellulaire sans glutamine, le procédé de culture comprenant :
la mise en culture d'une cellule mammifère dans le milieu de culture cellulaire exempt de glutamine dans des conditions permettant la survie ou la prolifération de la cellule,
dans lequel le milieu de culture cellulaire comprend de l'hypoxanthine à une concentration d'environ 150 µM à environ 750 µM et de la thymidine à une concentration d'environ 25 µM à environ 200 µM.

2. Procédé de culture selon la revendication 1, dans lequel la quantité d'ammoniac est réduite d'environ 10 % ou plus par rapport à celle contenue dans un milieu de culture sans glutamine qui ne comprend pas au moins l'un parmi l'hypoxanthine et la thymidine.

3. Procédé de culture selon l'une quelconque des revendications 1 et 2, dans lequel
(a) la cellule maintenue dans les conditions du procédé présente une consommation de glucose accrue par rapport à celle observée dans un milieu de culture sans glutamine qui ne comprend pas au moins l'un parmi l'hypoxanthine et la thymidine,
(b) la cellule maintenue dans les conditions du procédé présente une viabilité cellulaire accrue par rapport à celle observée dans un milieu de culture sans glutamine qui ne comprend pas au moins l'un des composés hypoxanthine et thymidine, ou
(c) la cellule maintenue dans les conditions du procédé présente un niveau d'expression (titre) accru d'un polypeptide, par rapport à celui observé dans un milieu de culture sans glutamine qui ne comprend pas au moins l'un parmi l'hypoxanthine et la thymidine.

4. Procédé de culture selon la revendication 3, dans lequel
(a) la consommation de glucose est augmentée d'environ 10 % ou plus, par rapport à la consommation de glucose dans un milieu de culture sans glutamine qui ne comprend pas au moins l'un parmi l'hypoxanthine et la thymidine,
(b) la viabilité cellulaire est augmentée d'environ 3 % ou plus, par rapport à la viabilité cellulaire dans un milieu de culture sans glutamine qui ne comprend pas au moins l'un parmi l'hypoxanthine et la thymidine, ou
(c) la cellule maintenue dans les conditions du procédé présente un niveau d'expression (titre) accru d'un polypeptide, par rapport à celui observé dans un milieu de culture exempt de glutamine qui ne comprend pas au moins l'un parmi l'hypoxanthine et la thymidine.

5. Procédé de culture selon l'une quelconque des revendications 1 à 4, comprenant en outre l'ajout d'au moins l'un parmi l'uridine et le magnésium au milieu de culture cellulaire.

6. Procédé de culture selon l'une quelconque des revendications 1 à 5, dans lequel la protéine cible est choisie parmi
(a) le groupe constitué d'un anticorps ou d'un fragment de celui-ci se liant à un antigène, d'une immunoadhésine, d'une molécule de signalisation de la superfamille du facteur de croissance transformant (TGF)-β, d'un facteur de coagulation sanguine, d'un anticorps anti-récepteur du facteur de nécrose tumorale (TNFR), d'un anticorps anti-récepteur 2 du facteur de croissance épidermique humain (HER2), et TNFR:Fc ; ou
(b) le groupe constitué par l'abagovomab, l'abciximab, l'adalimumab, l'adecatumumab, l'alemtuzumab, l'altumomab, l'altumomab pentétate, l'anatumomab, l'anatumomab mafenatox, l'arcitumomab, l'atlizumab, le basiliximab, le bectumomab, l'ectumomab, le belimumab, le benralizumab, le bevacizumab, brentuximab, canakinumab, capromab, capromab pendetide, catumaxomab, certolizumab, clivatuzumab tétraxétane, daclizumab, dénosumab, éculizumab, édrecolomab, éfalizumab, étaracizumab, ertumaxomab, fanolesomab, fontolizumab, gemtuzumab, girentuximab, golimumab, ibritumomab, igovomab, infliximab, ipilimumab, labetuzumab, mepolizumab, muromonab, muromonab-CD3, natalizumab, necitumumab, nimotuzumab, ofatumumab, omalizumab, oregovomab, palivizumab, panitumumab, ranibizumab, rituximab, satumomab, sulesomab, ibritumomab, ibritumomab tiuxetan, tocilizumab, tositumomab, trastuzumab, ustekinumab, visilizumab, votumumab, zalutumumab, brodalumab, anrukinzumab, bapineuzumab, dalotuzumab, demcizumab, ganitumab, inotuzumab, mavrilimumab, moxetumomab pasudotox, rilotumumab, sifalimumab, tanezumab, tralokinumab, tremelimumab, urelumab, adornase alfa, Rebif, becaplermin, alteplase, laronidase, alefacept, aflibercept, Raxibacumab, darbepoétine alfa, concentré de becaplermin, interféron bêta-1b, toxine botulique de type A, rasburicase, asparaginase, époétine alfa, étanercept, agalsidase bêta, interféron alfacon-1, interféron alfa-2a, anakinra, toxine botulique de type B, pegfilgrastim, oprelvekin, filgrastim, denileukin diftitox, peginterféron alfa-2a, aldesleukin, dornase alfa, interféron bêta-1a, becaplermin, reteplase, interféron alfa-2, tenecteplase, drotrecogin alfa, rilonacept, romiplostim, méthoxypolyéthylène glycol-époétine bêta, inhibiteur de la C1 estérase, idursulfase, alglucosidase alfa, abatacept, galsulfase, palifermin et interféron gamma-1b.

7. Procédé de culture selon l'une quelconque des revendications 5 et 6, dans lequel la cellule maintenue dans le milieu de culture cellulaire présente une viabilité cellulaire accrue, de préférence d'environ 3 % ou plus, par rapport à celle dans un milieu de culture sans glutamine qui ne comprend pas au moins l'un parmi l'uridine et le magnésium.

8. Procédé de culture selon la revendication 7, dans lequel la cellule maintenue dans le milieu de culture cellulaire présente une densité cellulaire viable accrue, de préférence d'environ 5 % ou plus, par rapport à celle dans un milieu de culture sans glutamine qui ne comprend pas au moins l'un parmi l'uridine et le magnésium.

9. Procédé de culture selon l'une quelconque des revendications 1 à 8, dans lequel la cellule est choisie parmi le groupe constitué des cellules CHO, DG44, HEK, NS0, PER.C6, HeLa et MDCK.

10. Procédé de culture selon l'une quelconque des revendications 1 à 9, dans lequel la culture comprend des conditions d'au moins un changement de température, des conditions d'au moins un changement de pH, ou une combinaison de ceux-ci,
dans lequel les conditions d'au moins un changement de température comprennent les processus suivants : une cellule est cultivée dans un milieu à une première température pendant 3 jours ou plus, la température est changée à une deuxième température, la deuxième température étant d'environ 1 °C à environ 8 °C inférieure à la première température, et la cellule est maintenue à la deuxième température pendant environ 2 jours ou plus, et
dans lequel les conditions d'au moins un changement de pH comprennent les processus suivants : une cellule est cultivée dans un milieu à une première valeur de pH pendant environ 2 jours ou plus, le pH est changé à une deuxième valeur de pH, la deuxième valeur de pH étant inférieure d'environ 0,05 à environ 1 à la première valeur de pH, et la cellule est cultivée à la deuxième valeur de pH pendant environ 1 jour ou plus.

11. Le processus de culture de l'une quelconque des revendications 1 à 10, comprenant en outre l'ajout de glucose au milieu de culture cellulaire sans glutamine de telle sorte que la concentration en glucose soit maintenue à environ 2 g/L ou plus.

12. Utilisation d'un milieu de culture cellulaire sans glutamine pour réduire la quantité d'ammoniac lorsqu'une protéine cible est produite dans le milieu de culture cellulaire sans glutamine,
dans lequel le milieu de culture cellulaire comprend de l'hypoxanthine à une concentration d'environ 150 µM à environ 750 µM et de la thymidine à une concentration d'environ 25 µM à environ 200 µM.
